Europäisches Patentamt

European Patent Office

Office européen des brevets

(19)

(11) Veröffentlichungsnummer: **0 011 809**
**A1**

(12) # EUROPÄISCHE PATENTANMELDUNG

(21) Anmeldenummer: **79104605.5**

(22) Anmeldetag: **20.11.79**

(51) Int. Cl.³: **A 61 F 1/00**

(30) Priorität: 22.11.78 DE 2850586

(43) Veröffentlichungstag der Anmeldung:
11.06.80 Patentblatt 80/12

(84) Benannte Vertragsstaaten:
AT BE CH FR GB IT LU NL SE

(71) Anmelder: **Battelle-Institut e.V.**
**Am Römerhof 35**
**D-6000 Frankfurt/Main 90(DE)**

(72) Erfinder: **Reiner, Roland, Dr.**
**Gehspitz 11**
**D-6236 Eschborn(DE)**

(72) Erfinder: **Biehl, Uwe**
**Saalburgallee 36**
**D-6000 Frankfurt/Main(DE)**

(74) Vertreter: **Blum, Klaus-Dieter, Dipl.-Ing.**
**Am Römerhof 35**
**D-6000 Frankfurt/Main 90(DE)**

(54) **Einlage zur Prothesenverankerung.**

(57) Die Einlage wird zur Prothesenverankerung in einem Knochenhohlraum benutzt und besteht aus einer bioinerten, bei guter Reißfestigkeit dehnbaren Polymerfolie, die entsprechend der Form des Knochenhohlraums geformt ist. Die Folie ist nicht durchlässig für den Knochenzement und für die Körperflüssigkeiten und enthält in einer Menge von 5-40 Gew.% bezogen auf das Gewicht des Polymeren, bioinerte und/oder bioresorbierbare Substanzen, z.B. natürliche Knochenspäne oder Claciumphosphate.

EP 0 011 809 A1

389-62/26/78
CASCH/DOJ                    17. November 1978


BATTELLE - INSTITUT E.V., Frankfurt (Main)


================================

Einlage zur Prothesenverankerung

================================


Die Erfindung betrifft eine Einlage zur Prothesenverankerung mittels eines Knochenzements in einem Knochenhohlraum bestehend aus einer entsprechend der Form des Knochenhohlraums geformten, knochenverträglichen Folie, die nicht durchlässig für den noch nicht ausgehärteten Knochenzement und für die Körperflüssigkeiten ist.

In der DE-PS 2 334 643 ist ein Hilfsmittel zum Befestigen einer Endoprothese mittels eines mineralischen Zementes in einem Knochenhohlraum beschrieben, die aus einer in biologischer Umgebung resorbierbaren Folie besteht. Diese Folie ist entsprechend der Form des Knochenhohlraumes, z.B. als Säckchen, geformt. Da die mineralischen Zemente den Nachteil haben, daß sie nur in trockener Umgebung abbinden, wird diese Folie in den Knochenhohlraum eingelegt, wo sie mindestens für die Dauer des Aushärtens des Zementes diesen Hohlraum gegenüber dem Knochen abdichtet, aus welchem Blut oder sonstige Körperflüssigkeiten austreten. Da die Folie resorbierbar ist, wird sie nach einer bestimmten Zeit vom Körper abgebaut. Der Nachteil einer solchen Folie ist jedoch, daß die Resorbierung lange Zeit nach dem Aushärten des Zementes erfolgt, so daß die Verankerung später nicht die erwünschte Festigkeit aufweist.

Die größte Zahl aller heute in Röhrenknochen implantierten Prothesen werden meist mit Kunststoff-Knochenzement, z.B. auf Basis Polymethacrylat, fixiert. Man verwendet im allgemeinen eine Mischung von Polymethacrylat mit monomerem Methylmethacrylat unter Zugabe der nötigen Polymerisationskatalysatoren. Ein wesentlicher Nachteil solcher Knochenzemente ist die besonders während der Aushärtung des Zementes stattfindende Diffusion von Monomer in das umliegende Gewebe, das dadurch zerstört wird. Ein weiterer Nachteil ist, daß gerade an der Zementgrenzfläche nicht optimale Polymerisationsbedingungen herrschen, wodurch schwer gut knochenverträgliche Grenzflächen erhalten werden können. Bei Zugabe von bioverträglichen Füllstoffen, z.B. Fasern und keramischen Pulvern zur Verbesserung der Knochenverträglichkeit, wird die Gesamtmasse des Zements mit diesen durchsetzt, was mechanische Nachteile mit sich bringt.

Der Erfindung liegt daher die Aufgabe zugrunde, eine Einlage zu entwickeln, die die Verwendung sowohl mineralischer als auch Kunststoff-Knochenzemente zum Befestigen einer Endoprothese in einem Knochenhohlraum ermöglicht, eine optimal bioverträgliche und bioaktive Grenzfläche zum Knochen ergibt, eine bessere Verankerung nach dem Aushärten des Zements mit dem Knochenhohlraum erlaubt und die Schädigung des umliegenden Gewebes durch Monomere verhindert.

Diese Aufgabe wird erfindungsgemäß durch eine Einlage gelöst, die aus einer bioinerten und bei guter Reißfestigkeit dehnbaren Polymerfolie besteht, in die 5 - 40 %, bezogen auf das Gewicht des Polymeren, bioinerte und/oder bioresorbierbare, knochenverträgliche Substanzen eingelagert sind.

Je nach dem, ob bioinerte oder bioresorbierbare Substanzen in das Polymerisat eingelagert werden, bleibt die erfindungsgemäße Einlage im Knochenhohlraum entweder vollständig erhalten oder wird sie nach dem Aushärten des Knochenzements durch Resorbierung der eingelagerten Substanzen teilweise durch einwachsenden neuen Knochen durchdrungen.

Bioinerte Polymerisate, die im Körper eingesetzt werden können sind bekannt ("Reviews in Macromolecular Chemistry", 1 (2), 355 - 391 1966 von D.J. Lymann; "Die Beständigkeit von Kunststoffen und Gummi", Prof. Dr. C.M. von Meyenbug, Carl Hauser Verlag, München-Wien, 1978). Wesentlich ist, daß sie knochenverträglich und die daraus hergestellten Folien bei guter Reißfestigkeit dehnbar sind. Solche Polymerisate sind z.B. Polyacryl- und Polymethacrylderivate, Derivate von Polyhydroxyverbindungen, Silikone, Polyester, Polyurethane, Polyamide, Polyäthylen, Polypropylen oder halogenierte, insbesondere fluorierte Polymerisate.

Die Flexibilität, Härte und Dehnbarkeit der Polymeren kann durch Zugabe von Weichmachern entsprechend eingestellt werden. Die Dehnung sollte vorzugsweise 20 bis 30 % ohne Reißen betragen. Die Weichmacher werden vorzugsweise in einer Menge von 10 - 25 Gew.-%, bezogen auf das Gewicht des Polymeren, verwendet. Die Auswahl des geeigneten Weichmachers ist nicht kritisch. Hierzu können übliche Stoffe, z.B. Glyzerin und Polyäthylenglykole, herangezogen werden.

Die in den Polymeren einzulagernden Substanzen bestehen aus bioinerten oder bioresorbierbaren knochenverträglichen Materialien, wie keramische Massen, natürliche Knochenspäne oder Calciumphosphat-Derivate. Gemäß einer bevorzugten Ausführungsform der Erfindung werden Calciumphosphate, die aus CaO und $P_2O_5$ im Mengenverhältnis von 2:2 bis 4:1 zusammengesetzt sind, verwendet. Insbesondere eignet sich gesintertes Tricalciumphosphat, das aus einer Mischung von $\alpha$- und ß-Modifikation, gegebenenfalls mit einer amorphen Phase besteht.

Die Einbringung dieser Substanzen in die Polymermasse kann in an sich bekannter Weise erfolgen. Hierbei können diese Substanzen als Granulate, Partikel oder in einer anderen Form verwendet werden. Wesentlich ist, daß sie insbesondere auf der dem Knochen zugewandten Oberfläche der Einlage wenigstens teilweise eine bioaktive Grenzfläche ergeben. Dies bedeutet, daß an dieser Fläche der Folie die eingelagerten Substanzen unbedeckt vom Polymermaterial, vorzugsweise in höherer Konzentration, vorliegen. Hierfür wird die dem Knochen zugewandte Oberfläche der Polymerfolie mit der anorganischen Substanz paniert oder die Partikel können nach der Einlagerung in das Polymerisat durch mechanische Bearbeitung, z.B. durch mechanisches Anschleifen bzw. Anschneiden der Folie, freigelegt werden. Es ist auch möglich, die äußere und innere Oberfläche der Einlage mechanisch oder chemisch zu behandeln, so daß einerseits das Knochenwachstum angeregt und andererseits eine bessere Veränkerung des Zements erzielt wird.

In die Folie können ferner pharmakologisch wirksame Mittel, z.B. Antibiotika, das Knochenwachstum stimulierende Substanzen, Seren usw. eingebracht werden.

Bei Verwendung von Kunststoff-Knochenzementen stellt die erfindungsgemäße Folie eine Diffusionsbarriere für das Monomer dar. Durch die resorbierbaren Anteile wird eine bessere Knochenverankerung erzielt. Wenn bioinerte Anteile in die Folie eingelagert sind, wird eine bessere Grenzfläche zwischen der Folie und dem Knochen erhalten.

In den nachfolgenden Ausführungsbeispielen wird die Herstellung der erfindungsgemäßen Folie erläutert.

Beispiel 1

20 Teile eines medizinisch reinen Polyurethan-Elastomers werden in 80 Teilen DMF gelöst. Von der homogenen Lösung wird ein ca. 200 µm dicker Film ausgestrichen bzw. ausgezogen. Auf den Film werden 2 Teile Pulver aus gesintertem Tricalciumphosphat (Korngröße ca. 100 µm) aufgestreut oder eingewalzt. Der erhaltene Film zeigt nach dem Trocknen sehr gute mechanische Eigenschaften und eine gute Flexibilität.

Beispiel 2 bis 4

Nach dem Verfahren des Beispiels 1 werden Folien aus folgenden Zusammensetzungen hergestellt. Die Dehnbarkeit der Folien ist durch die Variation der Menge an Polyäthylenglykol einstellbar.

24 Teile Vinylalkohol/Vinylacetat-Copolymere

6 Teile Polyäthylenglycol (MG 600)

1 Teil  Tricalciumphosphat

Toluol ad 100 Teile

10 Teile Polybutylmethacrylat

2.5 Teile Polyäthylenglycol (MG 600)

1 Teil   Tricalciumphosphat

Toluol ad 100 Teile


24 Teile Polyvinylalkohol/Polyvinylacetat-Copolymere

7,2 Teile Polyäthylenglycol (MG 600)

7,2 Teile Tricalciumphosphat

Toluol ad 100 Teile


Beispiel 5

Aus 9 Teilen medizinisch reiner Silikon-Gußmasse mit 1 Teil
Härtungsmittel (Catalyst M, Dow Corning 382) wird ein Film
ausgewalzt bzw. ausgezogen und mit 3 Teilen gesintertem Tricalciumphosphat bestreut. Der Film wird bei 120 $^{O}$C ausgehärtet und zeigt eine gute mechanische Stabilität, gute Flexibilität und hohe Dehnbarkeit.


Beispiel 6

Ein am Ende abgerundeter zylindrischer Dorn mit einem Durchmesser von 15 mm und einer Länge von 150 mm wird durch Tauchen
in eine Polymerlösung aus 20 Teilen Polyurethan in 80 Teile
DMF mit dem Polymer überzogen und anschließend mit Tricalciumphosphat-Pulver der Korngröße bis 60 um paniert, indem das
Pulver auf dem sich drehenden Dorn gestreut wird.


Beispiel 7

Eine Folie aus medizinisch reinem Polyäthylen wird bis zur Erweichungstemperatur erwärmt. Auf die warme Folie wird Tricalciumphosphat-Pulver aufgestreut und eingewalzt. Der erhaltene
Film zeigt gute mechanische Eigenschaften und eine gute Flexibilität.

389-62/26/78
CASCH/DOJ                         17. November 1978


BATTELLE - INSTITUT E.V., Frankfurt (Main)


Patentansprüche


1. Einlage zur Prothesenverankerung mittels eines Knochenzements in einem Knochenhohlraum bestehend aus einer entsprechend der Form des Knochenhohlraums geformten knochenverträglichen Folie, die nicht durchlässig für den noch
nicht ausgehärteten Knochenzement und für die Körperflüssigkeiten ist, dadurch gekennzeichnet, daß sie aus einer
bioinerten und bei guter Reißfestigkeit dehnbaren Polymerfolie besteht, in die 5 - 40 %, bezogen auf das Gewicht
des Polymeren bioinerte und/oder bioresorbierbare, knochenverträgliche Substanzen eingelagert sind.

0011809

2. Einlage nach Anspruch 1, <u>dadurch gekennzeichnet</u>, daß als eingelagerte Substanzen keramische Massen, natürliche Knochenspäne oder Calciumphosphat-Derivate, vorzugsweise gesinterte Calciumphosphate, die aus CaO und $P_2O_5$ im Mengenverhältnis von 2:1 bis 4:1 zusammengesetzt sind, verwendet werden.

3. Einlagen nach Anspruch 1 und 2, <u>dadurch gekennzeichnet</u>, daß als bioinerte Polymere medizinisch reine Polyacryl- und Polymethacryl-Derivate, Derivate von Polyhydroxyverbindungen, Silikone, Polyester, Polyurethane, Polyamide, Polyäthylen, Polypropylen oder halogenierte, insbesondere fluorierte, Polymere verwendet werden.

4. Einlage nach Anspruch 1 bis 3, <u>dadurch gekennzeichnet</u>, daß die Polymerisate 10 - 25 %, bezogen auf das Gewicht des Polymeren eines Weichmachers, z.B. Glyzerin und Polyäthylenglykol, enthalten.

5. Einlage nach Anspruch 1 bis 4, <u>dadurch gekennzeichnet</u>, daß die eingelagerten Substanzen auf der dem Knochen zugewandte Oberfläche der Einlage in höherer Konzentration vorliegen.

## EUROPÄISCHER RECHERCHENBERICHT

Europäisches
Patentamt

1 · 7, · ·

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| X | US - A - 3 892 649 (PHILIPS & SHAW)<br>* Zusammenfassung; Spalte 1, Zeilen 3-9; Spalte 2, Zeilen 9-37; Spalte 4, Zeile 9 - Spalte 5, Zeile 5; Spalte 5, Zeilen 25-51, Ansprüche 1,2 *<br>-- | 1-3 | A 61 : 1/00 |
| X | DE - B - 2 620 890 (BATTELLE)<br>* Spalte 1, Zeilen 4-21; Ansprüche 1,2 *<br>-- | 1-3 | |
| X | DE - B - 2 620 891 (BATTELLE)<br>* Spalte 1, Zeilen 3-10; Ansprüche *<br>-- | 1-3 | **RECHERCHIERTE SACHGEBIETE (Int. Cl. 3)**<br>A 61 F |
| | DE - A - 2 502 884 (HILDEBRANDT)<br>* Seite 3, Zeilen 1-23; Seite 4, Zeile 21 - Seite 5, Zeile 10; Seite 5, Zeilen 25-28; Seite 6, Zeilen 1-26; Ansprüche 1-3 *<br>-- | 1-3 | |
| | DE - A - 2 008 708 (HODOSH)<br>* Seite 11, Zeile 14 - Seite 12, Zeile 7 *<br>-- | 2-4 | **KATEGORIE DER GENANNTEN DOKUMENTE** |
| | DE - A - 2 518 153 (SULZER)<br>* Seite 9, Zeilen 1-10; Seite 10, Zeilen 13-15; Ansprüche 1,8 *<br>-- | 1-3 | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | DE - B - 2 501 583 (LEITZ) | 1-3,5 | |

Der vorliegende Recherchenbericht wurde für alle Patentansprüche erstellt.

&: Mitglied der gleichen Patentfamilie, übereinstimmendes Dokument

| Recherchenort | Abschlußdatum der Recherche | Prüfer |
|---|---|---|
| Den Haag | 05-02-1980 | PESCHEK |

EPA form 1503.1   06.78

BAD ORIGINAL

EP 79 10 4605

-2-

| | **EINSCHLÄGIGE DOKUMENTE** | | **KLASSIFIKATION DER ANMELDUNG (Int.Cl. [3])** |
| --- | --- | --- | --- |
| Kategorie | Kennzeichnung des Dokuments mit Angabe, soweit erforderlich, der maßgeblichen Teile | betrifft Anspruch | |
| | * Spalte 1, Zeilen 4-24; Ansprüche 1-3; Spalte 4, Zeilen 17-25; Spalte 6, Zeilen 9-14 * | | |
| | -- | | |
| | US - A - 3 740 769 (HABOUSH)<br>* Spalte 3, Zeile 67 - Spalte 4, Zeile 4; Spalte 4, Zeilen 29-58; Figur 3 * | 2 | |
| | -- | | **RECHERCHIERTE SACHGEBIETE (Int. Cl. [3])** |
| | US - A - 3 228 393 (MICHELE)<br>* Spalte 4, Zeilen 40-50 * | 2 | |
| | -- | | |
| | US - A - 3 053 251 (BLACK)<br>* Spalte 4, Zeilen 11-20 * | 2 | |
| | ---- | | |

EPA Form 1503.2  06.78